(19) Europäisches Patentamt / European Patent Office / Office européen des brevets

(11) **EP 3 660 498 B1**

(12) **EUROPEAN PATENT SPECIFICATION**

(45) Date of publication and mention
of the grant of the patent:
**07.08.2024 Bulletin 2024/32**

(21) Application number: **18208705.6**

(22) Date of filing: **27.11.2018**

(51) International Patent Classification (IPC):
*G01N 27/12* *(2006.01)* *G01N 33/00* *(2006.01)*
*G01N 15/06* *(2024.01)*

(52) Cooperative Patent Classification (CPC):
**G01N 27/121; G01N 15/06; G01N 27/12;**
**G01N 33/0016;** G01N 27/128; G01N 33/0006;
G01N 2015/0026; G01N 2015/0046

(54) **AMBIENT SENSOR WITH ACTUATOR**

UMGEBUNGSSENSOR MIT AKTUATOR

CAPTEUR ENVIRONNEMENTAL COMPORTANT UN ACTIONNEUR

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**03.06.2020 Bulletin 2020/23**

(73) Proprietor: **Sensirion AG**
**8712 Stäfa (CH)**

(72) Inventors:
• **Schumm, Johannes**
**8712 Stäfa (CH)**

• **Gerner, Pascal**
**8712 Stäfa (CH)**
• **Rüffer, Daniel**
**8712 Stäfa (CH)**
• **Niederberger, Dominik**
**8712 Stäfa (CH)**

(74) Representative: **Toleti, Martin**
**E.Blum & Co. AG**
**Vorderberg 11**
**8044 Zürich (CH)**

(56) References cited:
**US-A1- 2012 323 440    US-A1- 2014 134 053**
**US-A1- 2014 355 649    US-A1- 2018 143 084**

**Description**

Technical Field

The present invention relates to a sensor unit with a sensor for sensing a variable of an ambient gas and an actuator. A further aspect of the invention relates to a method for determining an ambient value of a variable in a gas in the ambience of such sensor unit.

Background Art

Sensors integrated into electronic devices are ubiquitous, e.g. in mobile phones, wearables, IoT (internet of things) devices, or conventional sensing devices. They sense a variety of physical quantities of the ambience, and in particular of an ambient gas such as air, another gas, an aerosol, or a dispersion. The physical quantities may include but are not limited to one or more of humidity, temperature, a concentration of a gas, or a concentration of particulate matter in the ambient gas.

The accuracy and reliability of measurements of such sensor may suffer from disturbances originating from the electronic device at least partly surrounding it. Common sources of disturbances may include one or more of a heat flow from components of the electronic device, outgassing or gas soaking from material of the electronic device, and others. Complex compensation algorithms have been developed in order to still return accurate and reliable values for the physical quantities in the ambience, i.e. outside of the electronic device. However, the performance of these algorithms is limited.

Apart from purely algorithmic approaches to improving the accuracy and reliability of measurements, it has been suggested earlier to enhance a flow of the ambient gas to and away from the sensor. For instance, US 2014/0134053 A1 discloses a portable electronic device with a housing, a chemical sensor arranged inside the housing and adapted to measure a property of at least one analyte, and a duct terminating at an opening in the housing for exposing the chemical sensor to the gas to be analyzed, and an actuator to generate a flow within the duct.

US 2012/323440 A1 refers to a method for determining ambient air temperature outside of a vehicle with a sensor mounted on the vehicle. The method includes the steps of determining a road speed of the vehicle, comparing the determined road speed to a reference road speed value, if the determined road speed is below the reference road speed value, activating a device to move ambient air across the sensor, the ambient air being drawn from a location outside the vehicle, taking a temperature reading with the sensor, and, if the temperature reading is lower than a stored temperature value, storing the lower temperature reading as the ambient air temperature.

US 2014/355649 A1 relates to a portable electronic device with one or more integrated temperature sensors for measuring an ambient temperature. A compensator is provided for reducing the difference between a sensor output and the ambient temperature with the compensator switching depending on environmental and/or handling conditions between a plurality of compensation modes selected dependent on measurements of one or more other integrated and/or external sensors.

US 2018/143084 A1 describes a method for processing a signal supplied by a sensor. The method comprises receiving the sensed signal and compensating the sensed signal for a contribution caused by one or more components thermally coupled to the sensor. The compensated signal is compensated in its dynamics, and the dynamics adjusted compensated sensor signal is provided.

It is an objective of the present invention to provide a sensor unit with an actuator that has an improved quality in the determination of the ambient value of the physical quantity in question.

Disclosure of the Invention

The problem of improving accuracy and reliability in the determination of the ambient value of the physical quantity is solved by a sensor unit according to claim 1 and a method according to claim 14. The sensor unit may e.g. be an IoT device, a mobile phone, a wearable or any other sensor device.

According to a first aspect of the invention, a sensor unit comprises a sensor for sensing a variable of an ambient gas, and an actuator for supplying the ambient gas to the sensor. Supplying the ambient gas shall include a direct supply of the ambient gas to the sensor, but shall also include an enhancement of the ambient gas in the vicinity of the sensor e.g. supported by other processes such as diffusion. The ambient gas may comprise one of a gas, an aerosol, or a dispersion, and may specifically refer to ambient air surrounding the sensor unit or to another ambient gas. Examples for the variable sensed by the sensor and representing the physical quantity to be measured include but are not limited to one or more of a humidity, a temperature, a gas concentration or a concentration of particulate matter of the ambient gas. Accordingly, the sensor may also be configured to detect solids or liquids comprised in a gas, such as in an aerosol. For example, the variable may refer to particulates in the ambient air, and the value of the variable may then refer to the concentration or the size of the particulate. The actuator may be a direct or indirect actuator, for instance include one or more of a fan, a pump, or a loudspeaker. In particular, the actuator may be electrically controllable. The invention may also be applied to devices which already comprise an actuator for different purposes, such as an air purifier with a fan. Further actuator arrangements are described in US 2014/0134053 A1. Further, the sensor unit comprises one or more components contributing to measurement results provided by the sensor such that a portion of the signal provided by the sensor stems from these one or more components rather than the ambience. As discussed in the background art section, such one or more components may cause a disturbance of a measurement of the sensor, e.g. through heat flow, outgassing or gas soaking of a material of the one or more components. For this reason, the sensor is assumed to provide a measurement result that is disturbed by such one or more components, i.e. the sensor provides a disturbed value of the variable to be sensed, while an undisturbed value of the variable of the ambience is desired to be known, which undisturbed value is referred to as ambient value in the following. Further, the sensor unit comprises an estimator for determining the ambient value of the variable absent the contribution of the one or more components. The ambient value is dependent on a first measurement result provided by the sensor in response to a first activation state of the actuator and a second measurement result provided by the sensor in response to a second activation state of the actuator, which second activation state is different from the first activation state. An advantage of the invention is that it allows an improved determination of the ambient value of the variable, i.e. a more accurate and more reliable ambient value of the variable.

In one embodiment, the first activation state may be an off state of the actuator and the second activation state may be an on state of the actuator. In another embodiment, the first activation state may be a low power state of the actuator and the second activation state may be a high power state of the actuator exceeding the power consumed by the actuator in the low power state. Hence, the first activation state and the second activation state preferably differ in a flow of ambient gas which flow is caused or is supported to and/or away from the sensor. In this way, preferably the values of the variable sensed at the sensor under the first activation state and the second activation state of the actuator differ. Under the assumption that the ambient value of the variable in the ambient gas as well as the contribution of the one or more components are equal for the first measurement result and the second measurement result, the ambient value of the variable may be derived.

Besides, the sensor unit may preferably comprise a channel with an inlet from and an outlet to the ambience, wherein the sensor is arranged in or at the channel for sensing the variable in the ambient gas passing the sensor. The actuator is advantageously arranged to supply the ambient gas from the inlet through the channel to the outlet thereby passing the sensor. For typical arrangements of actuators and channels it is again referred to the explanation in US 2014/0134053 A1. The estimator is adapted to monitor the activation state of the actuator and to accept two measurement results provided by the sensor as the first and the second measurement result if a corresponding second activation state deviates from a corresponding first activation state by a minimum margin, preferably by a minimum margin in power. In particular, the estimator is adapted to accept the two measurement results as the first and the second measurement result only if in addition a time interval between the corresponding first and the corresponding second measurement is less than a threshold value. Accepting a measurement result in this context includes applying the corresponding measurement results in the determination of the ambient value.

The condition on the time interval is supposed to ensure that the assumptions of a constant ambient value of the variable and a constant contribution of the one or more components to the measurement results are fulfilled. The assumptions may also be considered to be fulfilled if changes in the ambient value and the contribution of the one or more components are expected to remain within a defined range within the time interval or between the defined points in time, respectively.

The estimator is preferably configured to determine the ambient value of the variable absent the contribution of the one or more components based on a flux of the physical quantity represented by the variable from the one or more components to the sensor equating a flux the physical quantity represented by the variable from the sensor to the ambience for each of the first and the second activation state of the actuator. In particular the estimator is configured to determine the ambient value of the variable absent the contribution of the one or more components by solving equations derived from the continuity equation (eq1)

$$\frac{\partial c}{\partial t} + \nabla \cdot \mathbf{j} = R$$

wherein c is the variable or quantity of interest, e.g. a species concentration for mass transfer or heat for heat transfer, j is a total flux and R is a net volumetric source for c. For the first and second measurements, steady state may be assumed, $\partial c/\partial t = 0$, as well as that there are no sources or sinks in a volume under consideration, i.e. a small volume around the sensor. Hence the continuation equation simplifies to (eq2)

$$\nabla \cdot \mathbf{j} = \mathbf{0} \, .$$

A solution to the simplified continuity equation is that the total flux j, which is the sum of the diffusive flux $j_{diff}$ and the advective flux $j_{adv}$, also named convective flux, into and out of the volume is zero (eq3) :

$$\mathbf{j} = j_{diff} + j_{adv} = -\mathbf{D}\,\nabla\mathbf{c} + \mathbf{v}\mathbf{c} = \mathbf{0}$$

wherein D is a diffusivity, also named diffusion constant, e.g. mass diffusivity for mass transfer or thermal diffusivity for heat transport, and v is the velocity field the quantity c is moving with. Depending on the variable sensed by the sensor, e.g. temperature T or concentration c, such as humidity $c_{H20}$, gas concentration $c_{gas}$, or particulate matter concentration $c_{PM}$, the above equation of total flux further reduces to a simpler equation. This simpler equation may then be used to derive a ambient value of the variable in the ambience provided the first and second measurement results of the sensor are known, and provided transport and diffusive properties depending on the velocity v and the diffusivity D have been calibrated.

In an embodiment of the sensor unit, the variable is a temperature of the ambient air, the sensor is a temperature sensor, and the one or more components include electronic components emitting heat in response to being powered. In that case, the above equation eq3 may be written as (eq4)

$$\dot{Q}_{heat} - \dot{Q}_{amb} = 0$$

for the heat flux from the components emitting heat to the temperature sensor, which predominantly is a conductive heat flux, and the heat flux from the temperature sensor to the ambience, which depending on the activation state of the actuator may either be predominantly diffusive (e.g. actuator off) or convective (e.g. actuator on). With the temperature $T_{heat}$ of the at least one component emitting heat, the temperature $T_{SHT}$ of the temperature sensor, the temperature $T_{amb}$ of the ambience, the parameter $\alpha_{fan}$ of heat transport from the temperature sensor to the ambience, and the parameter $\beta$ of heat transport from the components emitting heat to the temperature sensor, equation eq4 may be rewritten as (eq5)

$$\alpha_{fan[1,2]} \left( T_{SHT[1,2]} - T_{amb} \right) = \beta \left( T_{heat} - T_{SHT[1,2]} \right)$$

Parameter $\beta$ is an effective parameter depending on a thermal conductivity or diffusivity, respectively, of material between the components emitting heat and the temperature sensor, but independent of the convective flow effected by the actuator. Parameter $\alpha_{fan}$ also is an effective parameter. If the actuator, e.g. a fan, is switched off, $\alpha_{fan}$ predominantly depends on a thermal diffusivity of material between the temperature sensor and the ambience, e.g. air in the channel and/or a casing of the sensor unit. If the actuator is switched on, $\alpha_{fan}$ predominantly depends on parameter of the flow of the ambient gas to and from the temperature sensor effected by the actuator, i.e. on the velocity v in equation eq3. Hence $\alpha_{fan}$ in particular has different values $\alpha_{fan1}$ and $\alpha_{fan2}$ for the actuator being switched on or off, or in low or high power mode, respectively.

Equation eq5 may be solved as a system of equations to yield the ambient value of the ambient temperature $T_{amb}$, if at least two measurements are performed under a first and a second activation state of the actuator, and if the effective parameters $\alpha_{fan1}$, $\alpha_{fan2}$ and $\beta$ are known, e.g. from calibration measurements. By performing more than two measurements at different activation states of the actuator, an optimization problem may be solved on the system of equations, resulting in an even more reliable ambient value of the ambient temperature.

In a different embodiment of the sensor unit, the variable is humidity of the ambient air, the sensor is a humidity sensor, and the one more components include a component releasing or absorbing humidity. The one or more components in particular include a component of a housing of the sensor unit.

In yet another embodiment of the sensor unit, the variable is a gas concentration in the ambient air, the sensor is a gas sensor sensitive to the gas to be sensed, and the one or more components include a component releasing or absorbing the gas the sensor is sensitive to. These one or more components in particular include a component of a housing of the sensor unit, and in particular the gas concentration is a concentration of volatile organic compounds, and the gas sensor is sensitive to the volatile organic compounds. A similar sensor may include a sensor sensing the concentration of particulate matter, which the above described derivation is also applicable to.

For the embodiments wherein the sensor senses humidity, a gas concentration, or any concentration c of any species in general, the transport processes to and from the sensor may be predominantly diffusive, if the actuator is switched off, relying on diffusion processes in the ambient gas within the channel itself. If the actuator is switched on, the transport of the species is effected predominantly by convection. Again, equation eq3 is valid in a small volume around the sensor (eq6)

$$j_{diff} + j_{adv} = 0$$

In analogy to equation eq5 for heat transport, equation eq6 may be rewritten in terms of effective parameters $\alpha_{fan1}$, $\alpha_{fan2}$ and $\beta$ (eq7) :

$$\alpha_{fan[1,2]} \left( c_{SHT[1,2]} - c_{amb} \right) = \beta \left( c_{mat} - c_{SHT[1,2]} \right)$$

wherein $c_{SHT}$ is a concentration of the species at the sensor, $c_{amb}$ is a concentration of the species in the ambience, and $c_{mat}$ is a concentration of the species resulting from components of the sensor unit. The concentration $c_{mat}$ may be due to soaking processes in case of humidity measurements, or due to outgassing in case of gas concentration measurements, e.g. VOC.

As described above for equation eq5, equation eq7 may be used to construct a system of equations for at least two measurements under different activation states of the actuator. The effective parameters $\alpha_{fan1}$, $\alpha_{fan2}$ and $\beta$ may either be assumed as known from calibration measurements, or they may be derived from the system of equations provided that enough measurements are taken.

In another embodiment, the underlying principle of the invention may further be exploited to perform a self-calibration of a sensor, e.g. a temperature sensor. During a start-up phase of the sensor unit, it is assumed that $T_{SHT} = T_{amb}$, such that system parameters, i.e. relevant parameters of heat transport such as $\alpha$ and $\beta$ may be calibrated.

In a further embodiment, the estimator of the sensor unit is used to eliminate cross-sensitivities between disturbing contributions originating from components and the different sensors, e.g. a temperature-dependence of a gas sensor. Yet a further embodiment of the sensor unit comprises two temperature sensors, in particular at different locations in the channel. The two temperature sensors may be influenced by disturbing contributions of the component in a different manner since the flow advects more or less heat. This in turn may be used to make measurements of the ambient value $T_{amb}$ of temperature in the ambience more accurate and reliable. Further details concerning different embodiments and applications of the sensor unit are given in the detailed description of the drawings.

According to a second aspect of the invention, a method is provided for determining an ambient value of a variable in a gas in an ambience of a sensor unit, according to claim 14. The sensor unit is provided and comprises a sensor for sensing the variable, an actuator for supplying the ambient gas to the sensor and one or more components contributing to measurement results provided by the sensor. The method comprises the steps of receiving a first measurement result in response to a first activation state of the actuator, receiving a second measurement result in response to a second activation state of the actuator, and determining the ambient value of the variable absent the contribution of the one or more components dependent on the first and the second measurement result.

According to a further aspect of the invention, a computer program comprises computer program code means to cause a processing unit of an estimator of a sensor unit as described above to execute the estimator method steps according to the method of claim 14.

It is noted that embodiments described in relation to one aspect of the invention shall be considered as disclosed in connection with the one or more other aspects, too. Other advantageous embodiments are listed in the dependent claims as well as in the description below.

Brief Description of the Drawings

The embodiments defined above and further aspects, features and advantages of the present invention can also be derived from the examples of embodiments to be described hereinafter and are explained with reference to the drawings. In the drawings, it is illustrated in

Fig. 1 a schematic view of a sensor unit according to an embodiment of the present invention,
Fig. 2 a schematic view of a heat transport process assumed for deriving an ambient value of a temperature according to an embodiment of the present invention,
Fig. 3 a diagram of a sensed temperature depending on different heating states and different activation states of the actuator according to an embodiment of the present invention,
Fig. 4 a schematic view of a water vapour transport process assumed for deriving an ambient value of humidity in the ambience according to an aspect of the present invention, and in

Fig. 5 a schematic view of a transport process of volatile organic compounds (VOC) assumed for deriving an ambient value of VOC concentration in the ambience according to an aspect of the present invention.

Detailed Description of the Drawings

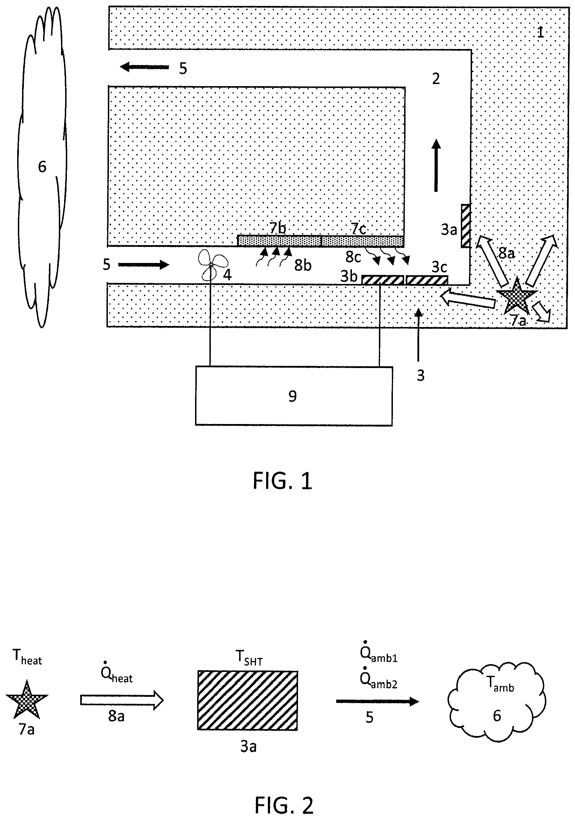

Fig. 1 shows a schematic view of a sensor unit according to an embodiment of the present invention. The sensor unit may e.g. be an IoT (internet of things) device, a mobile phone, an air purifier, a particulate matter module, or any other sensor device. It comprises a housing 1 and a channel 2 which is connected to an ambience 6 of the sensor unit, i.e. to an ambient gas outside of the sensor unit. At least one sensor 3 measures a variable of the ambient gas. The sensor 3 may include one or more of a temperature sensor 3a, a humidity sensor 3b, and a gas sensor 3c, the latter e.g. sensing the concentration of VOC. The sensor unit further comprises an actuator 4, e.g. a fan, or a piezo-electric crystal, which causes a flow 5 of the ambient gas through the channel 2 when activated. The actuator 4 preferably is controllable, e.g. electrically. It may have only two activation states, on and off, or it may be controllable into at least two on states, e.g. a low power and a high power mode. Different activation states of the actuator differ in the flow 5 of ambient gas which they cause in the channel 2, in particular in the flow velocity v. The different activation states may also be characterized by an amount of power consumed by the actuator 4, which preferably may be measured or controlled.

Further, the sensor unit comprises one or more components which disturb the measurements of sensors 3a-c, if all available. This may include a component 7a emitting heat which is transported to the sensors 3a and causes a measured value of a variable by the sensors 3a to differ from an ambient value of that variable in the ambience 6. Such component 7a may typically include one or more of a microcontroller, an RF module, a power converter, a display or a power supply. The transport of heat from the component 7a to the sensors 3a may be effected via heat conduction 8a, radiation or convection by a gas, of which conduction 8a is regarded as the predominant process. For the humidity sensor 3b, soaking processes 8b are particularly detrimental. Material 7b of the sensor unit - which material 7b may represent a component - may be soaked with water or water vapour, and in turn may release water or water vapour which reaches the sensor 3b, hence leading to wrong measurement results of an ambient humidity. For the gas sensor 3c, outgassing 8c of a component or a material 7c of the sensor unit tampers measurements of a true gas concentration in the ambience 6. Outgassing is understood as the release of a gas species from components or a material.

The sensor unit also comprises an estimator 9 which determines an ambient value of the sensed variable in the ambience 6. "Ambient value" means that a contribution of the components 7a, 7b or 7c disturbing the sensor measurements is absent. In order to cancel out these contributions, the estimator 9 uses measurement results provided by the one or more sensors 3a-c at different activation states of the actuator 4, in particular a first measurement result provided by the sensor 3 in response to a first activation state of the actuator 4 and a second measurement result provided by the sensor 3 in response to a second activation state of the actuator 4 different from the first activation state. This allows to eliminate the unknown contribution of the one or more components 7a-c. Preferably, the estimator 9 comprises a memory with values of relevant transport parameters for the measured variables and for the different activation states. The transport parameter are in particular known from calibration measurements.

For gaining measurement results of the sensors 3a-c at different activation states of the actuator 4, the estimator 9 may either monitor passively the actuator 9, e.g. via its power intake which may be measured or taken from power levels the actuator is tuned, and accept measurements only for certain activation states, i.e. if the power intake fulfills certain criteria.

In order to remove the unwanted contributions of one or more of the components 7a-c on the measurements of the one or more sensors 3a-c, at least two measurements at different activation states of the actuator 4 are required.

In the following, specific applications and the governing equations are presented for measurements of ambient temperature (example 1), ambient relative humidity (example 2), ambient total VOC concentration (example 3), and for a determination of a sensitivity of a gas sensor (example 4).

Example 1: Measurement of ambient temperature. Fig. 2 shows a schematic view of a heat transport process assumed for deriving an ambient value of an ambient temperature according to an embodiment of the present invention. It is desired to determine the ambient value of the temperature $T_{amb}$ in the ambience 6. However, the temperature sensor 3a measures the value $T_{SHT}$, which depends on $T_{amb}$, but also on the temperature $T_{heat}$ of component 7a emitting heat. It is assumed that $T_{heat}$ is unknown, but that $T_{heat}$, and hence the contribution of the component 7a, stays the same under varying activation states of the actuator 4, also called fan states. Also the temperature $T_{amb}$ in the ambience 6 is assumed to stay constant during the measurements at different fan states. The contribution of the component 7a is depicted in Fig. 2 via heat flux 8a which may be assumed to be predominantly conductive for most situations. The heat flux 8a ($\dot{Q}_{heat}$) from component 7a, a source of heat, to the temperature sensor 3a makes the measured temperature $T_{SHT}$ higher than $T_{amb}$. Hence the heat flux 8a contributes positively to an amount of heat contained in a small volume around the temperature sensor 3a, and hence to the temperature $T_{SHT}$. On the other hand, heat is transported out of the volume, thereby lowering the temperature $T_{SHT}$, predominantly by convective processes, i.e. by the flow 5 of ambient gas. This contribution is negative, and depends on the flow 5, in particular on its velocity v, which in turn depends on the activation state of the actuator 4: $\dot{Q}_{amb1} \neq \dot{Q}_{amb2}$ for different activation states of the actuator 4.

The situation of example 1 (Fig. 2) may be described by the heat equation as follows (eq8):

$$a_0 \frac{dT}{dt} = \sum_i \dot{Q}_i = -\dot{Q}_{amb} + \dot{Q}_{heat} = -\alpha_{fan}(T_{SHT} - T_{amb}) + \dot{Q}_{heat}$$
$$= -\alpha_{fan}(T_{SHT} - T_{amb}) + \beta(T_{heat} - T_{SHT})$$

wherein $a_0$ is a constant for reasons of dimensionality, and $\alpha_{fan}$ and $\beta$ are constant parameters indicative of heat transport properties for $\dot{Q}_{amb}$ and $\dot{Q}_{heat}$. In steady state with on & off fan states [1,2], one obtains (eq9)

$$\alpha_{fan[1,2]}(T_{SHT[1,2]} - T_{amb}) = \dot{Q}_{heat} = \beta(T_{heat} - T_{SHT[1,2]})$$

or as an equation system with the two fan states [1,2], i.e. on and off, and pre-characterized and constant parameters $\alpha_{fan1}$, $\alpha_{fan2}$ and $\beta$, (eq10)

$$\alpha_{fan1}(T_{SHT1} - T_{amb}) = \dot{Q}_{heat} = \beta(T_{heat} - T_{SHT1})$$

$$\alpha_{fan2}(T_{SHT2} - T_{amb}) = \dot{Q}_{heat} = \beta(T_{heat} - T_{SHT2})$$

Since $T_{amb}$ remains constant while fan is switched on and off, one gets two equations with unknown $\dot{Q}_{heat}$ and $T_{amb}$. This equation system can be solved for $\dot{Q}_{heat}$ to obtain the ambient temperature $T_{amb}$ (eq11)

$$T_{amb} = \frac{\alpha_{fan1}T_{SHT1} - \dot{Q}_{heat}}{\alpha_{fan1}} = \frac{\alpha_{fan2}T_{SHT2} - \dot{Q}_{heat}}{\alpha_{fan2}}$$

or (eq12)

$$T_{amb} = \frac{\alpha_{fan1}T_{SHT1} - \beta(T_{heat} - T_{SHT1})}{\alpha_{fan1}} = \frac{\alpha_{fan2}T_{SHT2} - \beta(T_{heat} - T_{SHT2})}{\alpha_{fan2}}$$

Under the given conditions, the temperature $T_{amb}$ in the ambience 6 may be determined from two measurement results of the temperature sensor 3a at different fan states (eq13) :

$$T_{amb} = F(T_{SHT1}, T_{SHT2}, \alpha_{fan1}, \alpha_{fan2}, \beta)$$

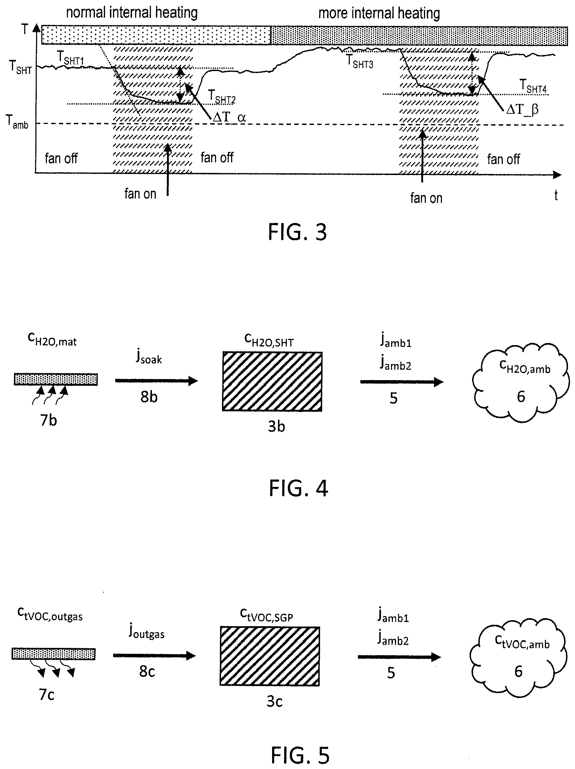

Fig. 3 shows a diagram of a sensed temperature $T_{SHT}$ depending on different heating states and different activation states (on, off) of the actuator, i.e. of the fan, according to an embodiment of the present invention. A time series of the sensed temperature $T_{SHT}$ is shown for normal internal heating, followed by more internal heating, all measurements being taken at constant ambient temperature $T_{amb}$. In the beginning, the fan is in an off state, and the sensed temperature $T_{SHT}$ varies around a constant value $T_{SHT1}$. Then, the fan is switched on, which leads to an enhanced heat transport away from the temperature sensor 3a, and hence at equal contribution of the component 7a, to a lower steady state $T_{SHT2}$. The lower value $T_{SHT2}$ is reached after a transition period, and is lower than $T_{SHT1}$ by a temperature difference $\Delta T$: $T_{SHT2} = T_{SHT1} - \Delta T\_\alpha$. When the fan is switched off again, the sensed temperature $T_{SHT}$ goes up to $T_{SHT1}$ again. In a second part of the diagram, there is more internal heating, i.e. a larger temperature $T_{heat}$ of component 7a. The more internal heating results in an increase of the sensed temperature $T_{SHT}$ to a value of $T_{SHT3}$, still at constant $T_{amb}$. When the fan is switched on, $T_{SHT}$ drops to $T_{SHT4} = T_{SHT3} - \Delta T\_\beta$. When the fan is switched off again, $T_{SHT}$ recovers to $T_{SHT3}$. $\Delta T$ may vary as is expressed in the present example by $\Delta T\_\alpha$ and $\Delta T\_\beta$, dependent on the amount of internal heating, which may vary over time.

With the time series of the sensed temperature $T_{SHT}$, Fig. 3 illustrates the effect of different activation states of the actuator 4, i.e. different fan states. At normal internal heating as well as at more internal heating, the sensed temperature $T_{SHT}$ drops by $\Delta T$ when the fan is switched on. In general, $T_{SHT}$ moves towards $T_{amb}$ when the fan is switched on since a heat exchange is enhanced by convective transport of heat, i.e. by the flow of the ambient gas. With pre-calibrated fan states, the value of the temperature difference $\Delta T$ may be used to estimate an accurate and reliable value of the ambient temperature $T_{amb}$ as described above.

Example 2: Measurement of ambient relative humidity. Fig. 4 shows a schematic view of a water vapour transport process assumed for deriving an ambient value of humidity in the ambience according to an aspect of the present invention. A material or a component 7b of the sensor unit is soaking and/or outgassing water and/or water vapour. This leads to a contribution $c_{H2C,mat}$ to the concentration $c_{H2O}$ of water vapour, i.e. to relative humidity, in channel 2 and at the humidity sensor 3b. The humidity value $c_{H2O,SHT}$ measured by the sensor 3b depends on a relative humidity $c_{H2O,amb}$ in the ambience 6 and on the soaked/outgassed humidity $c_{H2O,mat}$. Again starting from a consideration of fluxes, in this case a flux $j_{soak}$ of soaked/outgassed water vapour and a flux $j_{amb}$, the diffusion equation leads to the following formulation (eq14):

$$b_0 \frac{\partial c_{H_2O_{SHT}}}{\partial t} = \sum_i J_i = J_{soak} - J_{amb}$$
$$= \beta \left( c_{H_2O_{mat}} - c_{H_2O_{SHT}} \right) - \alpha \left( c_{H_2O_{SHT}} - c_{H_2O_{amb}} \right)$$

wherein $b_0$ is a dimensionality constant, and $\alpha$ and $\beta$ are constant parameters indicative of the water vapour transport, i.e. of the humidity transport, which may be calibrated beforehand. A steady state solution of equation eq14 with on and off fan states [1,2] yields (eq15)

$$\beta \left( c_{H_2O_{mat}} - c_{H_2O_{SHT}} \right) = \alpha \left( c_{H_2O_{SHT}} - c_{H_2O_{amb}} \right)$$

Two fan states [1,2], e.g. on and off, lead to two equations with two unknown parameters $c_{H2O,mat}$ and $c_{H2O,amb}$ (eq16)

$$\beta \left( c_{H_2O_{mat}} - c_{H_2O_{SHT1}} \right) = \alpha_{fan1} \left( c_{H_2O_{SHT1}} - c_{H_2O_{amb}} \right)$$

$$\beta \left( c_{H_2O_{mat}} - c_{H_2O_{SHT2}} \right) = \alpha_{fan2} \left( c_{H_2O_{SHT2}} - c_{H_2O_{amb}} \right)$$

This again means that a ambient value $c_{H2O,amb}$ may be determined as a function of the measured humidity value $c_{H2O,SHT}$ and the pre-calibrated parameters $\alpha_{fan1}$ $\alpha_{fan2}$ and $\beta$ (eq17) :

$$c_{H_2O_{amb}} = F(c_{H_2O_{SHT1}}, c_{H_2O_{SHT2}}, \alpha_{fan1}, \alpha_{fan2}, \beta)$$

It is possible that not only $\alpha$ but also the parameter $\beta$, which is characteristic for the humidity flux $j_{soak}$ towards the humidity sensor 3b, depends on the fan state. This may in particular be the case since a transport of water vapour or humidity may be assumed to be at least partly effected by convection, i.e. with the flow, when the fan is switched on. In that case, two different values $\beta_1$ and $\beta_2$ have to be inserted in the above equations eq16, respectively. The general principle and solution, however, does not change since both parameters $\beta_1$ and $\beta_2$ are assumed to be pre-calibrated. With the described method, an ambient value $c_{H2O,amb}$ in the ambience 6 may be determined from at least two measurement results $c_{H2O,SHT}$ of humidity at different fan states under the assumption that the contribution $c_{H2O,mat}$ of the soaking/outgassing component 7b as well as the ambient humidity $c_{H2O,amb}$ stay constant during the measurements.

Example 3: Measurement of ambient total volatile organic compound (tVOC) concentration. Fig. 5 shows a schematic view of a transport process of volatile organic compounds (VOC) assumed for deriving an ambient value of tVOC concentration in the ambience 6 according to an aspect of the present invention. A sensed concentration $c_{tVOC,SGP}$ is dependent on a ambient value $c_{tVOC,amb}$ of the tVOC concentration in the ambience 6 and a contribution $c_{tVOC,outgas}$ of a material or component 7c of the sensor unit outgassing VOC. The relevant fluxes of VOC species from and towards the VOC sensor 3c are again labelled $j_{amb}$ and $j_{outgas}$, respectively. The underlying principle of the transport process is the same as in example 2, hence all aspects of example 2 apply as well.

Accordingly, the diffusion equation with a dimensionality constant $c_0$ and parameters $\alpha$ and $\beta$ indicative of VOC species transport reads (eq18)

$$c_0 \frac{\partial c_{tVOC_{SGP}}}{\partial t} = \sum_i J_i = J_{outgas} - J_{amb}$$
$$= \beta \left( c_{tVOC_{outgas}} - c_{tVOC_{SGP}} \right) - \alpha \left( c_{tVOC_{SGP}} - c_{tVOC_{amb}} \right)$$

A steady state solution of equation eq18 has to fulfill (eq19)

$$\beta \left( c_{tVOC_{outgas}} - c_{tVOC_{SGP}} \right) = \alpha \left( c_{tVOC_{SGP}} - c_{tVOC_{amb}} \right)$$

For two fan states [1,2], e.g. on and off, equation eq18 leads to two equations with two unknown parameters (eq20)

$$\beta \left( c_{tVOC_{outgas}} - c_{tVOC_{SGP1}} \right) = \alpha_{fan1} \left( c_{tVOC_{SGP1}} - c_{tVOC_{amb}} \right)$$

$$\beta \left( c_{tVOC_{outgas}} - c_{tVOC_{SGP2}} \right) = \alpha_{fan2} \left( c_{tVOC_{SGP2}} - c_{tVOC_{amb}} \right)$$

By solving the equation system eq20 the ambient value $c_{tVOC,amb}$ of tVOC concentration in the ambience 6 may be determined as a function of the following measurements results and parameters (eq21):

$$c_{TVOC_{amb}} = F(c_{tVOC_{SGP1}}, c_{tVOC_{SGP2}}, \alpha_{fan1}, \alpha_{fan2}, \beta )$$

Similar to the applications of examples 1-3, the sensor unit and method of the invention may be applied to other signals and sensors, in particular other concentration measurements, e.g. a particulate matter sensor such as PM2.5.

Example 4: Determination of a sensitivity n of a gas sensor, e.g. a metal oxide (MOX) gas sensor. The inventive principle of performing measurements with a sensor 3 at different activation states of an actuator 4 can also be used to determine the sensitivity n of a MOX sensor.

The relationship between an electrical resistance R and a local gas concentration $c_{local}$ at the MOX sensor, which local gas concentration according to the glossary of example 3 is $c_{local} = c_{tVOC,SGP}$ , is as follows (eq22)

$$R = a\, c_{local}^n = a(c + c_B)^n,$$

wherein n is the sensitivity, a is a constant parameter, c is the concentration of the gas in the ambience, i.e. according to the glossary of example 3 is $c = c_{tVOC,amb}$, and $c_B$ is a concentration of the gas stemming from the one or more components of the device, i.e. the disturbing concentration, i.e. according to the glossary of example 3 is $c_B = c_{tVOC,outgas}$. Since the sensitivity n may vary in time, it is desired to estimate the sensitivity n during sensor operation in order to improve sensor accuracy and reliability.

In case the ambient concentration c is small enough, i.e. it may be assumed to be zero c=0, then the background concentration $c_B$ can be modified by an activation state of the actuator 4, e.g. a fan speed, such that the effective concentration $c_{local}$ is changed (eq23):

$$R_1 = a\, c_B^n$$

If the fan lowers the background concentration by a factor *f* that was pre-characterized and remains constant over time, one obtains (eq24)

$$R_2 = a \left( \frac{c_B}{f} \right)^n$$

It follows that the sensitivity of the gas-sensor can be determined by (eq25)

$$\frac{R_1}{R_2} = f^n$$

Further examples: The underlying principle of the invention may further be exploited to perform a self-calibration of a temperature sensor 3a. During a start-up phase of the sensor unit, it is assumed that $T_{SHT} = T_{amb}$, such that system parameters, i.e. relevant parameters of heat transport such as $\alpha$ and $\beta$ may be calibrated.

In a further embodiment, the estimator 9 of the sensor unit of Fig. 1 is used to eliminate cross-sensitivities between disturbing contributions 8a-c originating from components 7a-c and the different sensors 3a-c, e.g. a temperature-dependence of the tVOC sensor 3c.

Yet a further embodiment of the sensor unit comprises two temperature sensors 3a, in particular at different locations in the channel 2. The two temperature sensors 3a may be influenced by contributions of the component 7a in a different manner since the flow 5 advects more or less heat. This in turn may be used to make measurements of the ambient value $T_{amb}$ of temperature in the ambience 6 more accurate and reliable.

**Claims**

1. Sensor unit, comprising

   a sensor (3) for sensing a variable of an ambient gas,
   an actuator (4) for supplying the ambient gas to the sensor (3), and
   one or more components (7) contributing to measurement results provided by the sensor (3), **characterized by** an estimator (9) adapted for determining an ambient value of the variable absent the contribution of the one or more components (7) dependent on a first measurement result provided by the sensor (3) in response to a first activation state of the actuator (4) and a second measurement result provided by the sensor (3) in response to a second activation state of the actuator (4) different from the first activation state,
   wherein the estimator (9) is adapted to monitor the activation state of the actuator (3) and to accept two measurement results provided by the sensor (3) as the first and the second measurement result if a corresponding second activation state deviates from a corresponding first activation state by a minimum margin.

2. Sensor unit according to claim 1,
   wherein the estimator (9) is adapted to monitor the activation state of the actuator (3) and to accept the two measurement results provided by the sensor (3) as the first and the second measurement result if a corresponding second activation state deviates from a corresponding first activation state by a minimum margin in power.

3. Sensor unit according to claim 1 or claim 2,
   wherein the estimator (9) is adapted to accept the two measurement results as the first and the second measurement result only if in addition a time interval between the corresponding first and the corresponding second measurement is less than a threshold value.

4. Sensor unit according to claim 3,
   wherein the time interval is dimensioned such that the ambient value of the variable and the contribution of the one or more components (7) is expected to remain unchanged within the time interval or is expected to remain within a defined range within the time interval.

5. Sensor unit according to any of the preceding claims,
   wherein the first activation state is an off state of the actuator (4) and wherein the second activation state is an on state of the actuator (4).

6. Sensor unit according to any of the preceding claims 1 to 4,
   wherein the first activation state is a low power state of the actuator (4) and wherein the second activation state is a high power state of the actuator (4) exceeding the power consumed by the actuator (4) in the low power state.

7. Sensor unit according to any of the preceding claims,

   wherein the actuator (4) is an actuator electrically controllable into at least the first and the second activation state,
   wherein the actuator includes one or more of a fan, a pump or a loudspeaker.

8. Sensor unit according to any of the preceding claims,

   wherein the sensor (3) is configured to sense the variable of the ambient gas including one of a gas, an aerosol or a dispersion,
   in particular wherein the sensor (3) is configured to sense the variable of the ambient gas being ambient air surrounding the sensor unit.

9. Sensor unit according to any of the preceding claims,

   comprising a channel (2) with an inlet from and an outlet to the ambience (6),
   wherein the sensor (3) is arranged in or at the channel (2) for sensing the variable in the ambient gas, and
   wherein the actuator (4) is arranged to supply the ambient gas from the inlet through the channel (2) to the outlet thereby passing the sensor (3).

10. Sensor unit according to claim 8,

    wherein the variable is a temperature ($T_{amb}$) of ambient air,
    wherein the sensor (3) is a temperature sensor,
    wherein the one or more components (7) include an electronic component emitting heat in response to being powered.

11. Sensor unit according to claim 8,

    wherein the variable is humidity ($c_{H2O,amb}$) of ambient air,
    wherein the sensor (3) is a humidity sensor,
    wherein the one more components (7) include a component releasing or absorbing humidity,
    in particular wherein the one or more components (7) include a component of a housing (1) of the sensor unit.

12. Sensor unit according to claim 8,

    wherein the variable is a gas concentration ($c_{amb}$) in ambient air,
    wherein the sensor (3) is a gas sensor sensitive to the gas to be sensed,
    wherein the one or more components (7) include a component releasing or absorbing the gas the sensor is sensitive to,
    in particular wherein the one or more components (7) include a component of a housing (1) of the sensor unit,
    in particular wherein the gas concentration is a concentration of volatile organic compounds ($c_{tVOC,amb}$), and wherein the gas sensor is sensitive to the volatile organic compounds,
    in particular wherein the gas sensor is a metal oxide gas sensor and wherein the estimator is configured to determine a sensitivity of the metal oxide gas sensor dependent on the first measurement result and the second measurement result.

13. Sensor unit according to any of the preceding claims,

    wherein the estimator (9) is configured to determine the ambient value $c_{amb}$ of the variable absent the contribution $c_{mat}$ of the one or more components (7) based on a flux of the variable from the one or more components (7) to the sensor (3) equating a flux of the variable from the sensor (3) to the ambience (6), for each of the first and the second activation state of the actuator (4), and
    in particular wherein the estimator (9) is configured to determine the ambient value $c_{amb}$ of the variable absent the contribution $c_{mat}$ of the one or more components by solving the equation

$$\alpha_{[1,2]}\left(c_{SHT[1,2]} - c_{amb}\right) = \beta\left(c_{mat} - c_{SHT[1,2]}\right)$$

    wherein $c_{SHT}$ is a measurement result provided by the sensor (3), $\alpha$ is a parameter indicative of the flux of the variable from the sensor (3) to the ambience (6), and $\beta$ is a parameter indicative of the flux of the variable from the one or more components (7) to the sensor (3).

14. Method for determining an ambient value ($c_{amb}$) of a variable in a gas in the ambience (6) of a sensor unit, the method comprising the steps of:

providing a sensor unit comprising a sensor (3) for sensing the variable, an actuator (4) for supplying the ambient gas to the sensor (3), and one or more components (7) contributing to measurement results ($c_{SHT}$) provided by the sensor (3), **characterized by** an estimator (9) adapted for determining an ambient value of the variable absent the contribution of the one or more components (7),

the estimator (9) monitoring an activation state of the actuator (3) and accepting two measurement results provided by the sensor (3) as the first and the second measurement result if a corresponding second activation state deviates from a corresponding first activation state by a minimum margin, wherein the first measurement result ($c_{SHT1}$) is received in response to the first activation state of the actuator (4), and wherein the second measurement result ($c_{SHT2}$) is received in response to the second activation state of the actuator (4), and

the estimator (9) determining the ambient value ($c_{amb}$) of the variable absent the contribution ($c_{mat}$) of the one or more components (7) dependent on the first measurement result ($c_{SHT1}$) and the second measurement result ($c_{SHT2}$).

15. Computer program comprising computer program code means to cause a processing unit of the estimator (9) of the sensor unit of claim 1 to execute the estimator method steps according to claim 14.

**Patentansprüche**

1. Sensoreinheit, umfassend

einen Sensor (3) zum Erfassen einer Variable eines Umgebungsgases,
ein Aktor (4) zum Zuführen des Umgebungsgases zum Sensor (3), und
eine oder mehrere Komponenten (7), die zu vom Sensor (3) gelieferten Messergebnissen beitragen,
**gekennzeichnet durch** einen Schätzer (9), ausgestaltet zum Bestimmen eines Umgebungswerts der Variablen ohne den Beitrag der einen oder mehreren Komponenten (7) in Abhängigkeit von einem ersten Messergebnis, das vom Sensor (3) in Reaktion auf einen ersten Aktivierungszustand des Aktors (4) geliefert wird, und einem zweiten Messergebnis, das vom Sensor (3) als Antwort auf einen zweiten Aktivierungszustand des Aktors (4), der sich von dem ersten Aktivierungszustand unterscheidet, geliefert wird,
wobei der Schätzer (9) derart ausgestaltet ist, dass er den Aktivierungszustand des Aktors (3) überwacht und zwei vom Sensor (3) gelieferte Messergebnisse als das erste und das zweite Messergebnis zu akzeptieren, wenn ein entsprechender zweiter Aktivierungszustand von einem entsprechenden ersten Aktivierungszustand um eine minimale Spanne abweicht.

2. Sensoreinheit nach Anspruch 1,
wobei der Schätzer (9) derart ausgestaltet ist, dass er den Aktivierungszustand des Aktors (3) überwacht und die beiden vom Sensor (3) gelieferten Messergebnisse als erstes und zweites Messergebnis zu akzeptieren, wenn ein entsprechender zweiter Aktivierungszustand von einem entsprechenden ersten Aktivierungszustand um eine minimale Leistungsspanne abweicht.

3. Sensoreinheit nach Anspruch 1 oder Anspruch 2,
wobei der Schätzer (9) derart ausgestaltet ist, dass er die beiden Messergebnisse nur dann als erstes und zweites Messergebnis akzeptiert, wenn zusätzlich ein Zeitintervall zwischen der entsprechenden ersten und der entsprechenden zweiten Messung kleiner als ein Schwellenwert ist.

4. Sensoreinheit nach Anspruch 3,
wobei das Zeitintervall so bemessen ist, dass erwartet wird, dass der Umgebungswert der Variablen und der Beitrag der einen oder mehreren Komponenten (7) innerhalb des Zeitintervalls unverändert bleibt oder dass erwartet wird, dass er innerhalb des Zeitintervalls in einem definierten Bereich bleibt.

5. Sensoreinheit nach einem der vorangehenden Ansprüche,
wobei der erste Aktivierungszustand ein Aus-Zustand des Aktors (4) ist und wobei der zweite Aktivierungszustand ein Ein-Zustand des Aktors (4) ist.

6. Sensoreinheit nach einem der vorangehenden Ansprüche 1 bis 4,
wobei der erste Aktivierungszustand ein Zustand niedriger Leistung des Aktors (4) ist und wobei der zweite Aktivierungszustand ein Zustand hoher Leistung des Aktors (4) ist, der die vom Aktor (4) im Zustand niedriger Leistung verbrauchte Leistung übersteigt.

**7.** Sensoreinheit nach einem der vorangehenden Ansprüche,

wobei der Aktor (4) ein Aktor ist, der zumindest in den ersten und den zweiten Aktivierungszustand elektrisch steuerbar ist,
wobei der Aktor einen oder mehrere von einem Lüfter, einer Pumpe oder einem Lautsprecher umfasst.

**8.** Sensoreinheit nach einem der vorangehenden Ansprüche,

wobei der Sensor (3) so konfiguriert ist, dass er die Variable des Umgebungsgases einschliesslich eines Gases, eines Aerosols oder einer Dispersion erfasst,
insbesondere wobei der Sensor (3) so konfiguriert ist, dass er die Variable des Umgebungsgases erfasst, das die die Sensoreinheit umgebende Umgebungsluft ist.

**9.** Sensoreinheit nach einem der vorangehenden Ansprüche,

umfassend ein Kanal (2) mit einem Einlass von und einem Auslass in die Umgebung (6),
wobei der Sensor (3) in oder an dem Kanal (2) angeordnet ist, um die Variable im Umgebungsgas zu erfassen, und
wobei der Aktor (4) so angeordnet ist, dass er das Umgebungsgas vom Einlass durch den Kanal (2) zum Auslass leitet und dabei den Sensor (3) passiert.

**10.** Sensoreinheit nach Anspruch 8,

wobei die Variable eine Temperatur ($T_{amb}$) der Umgebungsluft ist,
wobei der Sensor (3) ein Temperatursensor ist,
wobei die eine oder die mehreren Komponenten (7) ein elektronisches Bauteil umfassen, das in Reaktion auf seine Stromversorgung Wärme abgibt.

**11.** Sensoreinheit nach Anspruch 8,

wobei die Variable die Feuchtigkeit ($c_{H2O,amb}$) der Umgebungsluft ist,
wobei der Sensor (3) ein Feuchtigkeitssensor ist,
wobei die eine oder mehreren Komponenten (7) eine feuchtigkeitsabgebende oder -aufnehmende Komponente umfassen,
insbesondere wobei die eine oder mehreren Komponenten (7) eine Komponente eines Gehäuses (1) der Sensoreinheit umfassen.

**12.** Sensoreinheit nach Anspruch 8,

wobei die Variable eine Gaskonzentration ($c_{amb}$) in der Umgebungsluft ist,
wobei der Sensor (3) ein Gassensor ist, der für das zu erfassende Gas empfindlich ist,
wobei die eine oder die mehreren Komponenten (7) eine Komponente umfassen, die das Gas, für das der Sensor empfindlich ist, freisetzt oder absorbiert,
insbesondere wobei die eine oder die mehreren Komponenten (7) eine Komponente eines Gehäuses (1) der Sensoreinheit umfassen,
insbesondere wobei die Gaskonzentration eine Konzentration von flüchtigen organischen Verbindungen ($c_{tVOC,amb}$) ist, und wobei der Gassensor für die flüchtigen organischen Verbindungen empfindlich ist,
insbesondere wobei der Gassensor ein Metalloxidgassensor ist und wobei der Schätzer konfiguriert ist, um eine Empfindlichkeit des Metalloxidgassensors in Abhängigkeit vom ersten Messergebnis und vom zweiten Messergebnis zu bestimmen.

**13.** Sensoreinheit nach einem der vorangehenden Ansprüche,

wobei der Schätzer (9) konfiguriert ist, den Umgebungswert $c_{amb}$ der Variablen ohne den Beitrag $c_{mat}$ der einen oder mehreren Komponenten (7) auf der Grundlage eines Flusses der Variablen von der einen oder mehreren Komponenten (7) zum Sensor (3) zu bestimmen, der einem Fluss der Variablen vom Sensor (3) zur Umgebung (6) für den ersten und zweiten Aktivierungszustand des Aktors (4) entspricht, und insbesondere wobei der Schätzer (9) so konfiguriert ist, dass er den Umgebungswert $c_{amb}$ der Variablen ohne den Beitrag $c_{mat}$ der einen oder mehreren Komponenten durch Lösen der Gleichung

$$\alpha_{[1,2]}(c_{SHT[1,2]} - c_{amb}) = \beta(c_{mat} - c_{SHT[1,2]})$$

wobei $c_{SHT}$ ein vom Sensor (3) geliefertes Messergebnis ist, $\alpha$ ein Parameter ist, der den Fluss der Variablen vom Sensor (3) zur Umgebung (6) anzeigt, und $\beta$ ein Parameter ist, der den Fluss der Variablen von der einen oder mehreren Komponenten (7) zum Sensor (3) anzeigt.

14. Verfahren zur Bestimmung eines Umgebungswertes ($c_{amb}$) einer Variablen in einem Gas in der Umgebung (6) einer Sensoreinheit, wobei das Verfahren die folgenden Schritte umfasst:

Bereitstellen einer Sensoreinheit mit einem Sensor (3) zum Erfassen der Variablen, eines Aktors (4) zum Zuführen des Umgebungsgases zum Sensor (3) und einer oder mehreren Komponenten (7), die zu den vom Sensor (3) gelieferten Messergebnissen ($c_{SHT}$) beitragen,
**gekennzeichnet durch** einen Schätzer (9), der zum Bestimmen eines Umgebungswertes der Variablen ohne den Beitrag der einen oder mehreren Komponenten (7) ausgestaltet ist,
wobei der Schätzer (9) einen Aktivierungszustand des Aktors (3) überwacht und zwei vom Sensor (3) gelieferte Messergebnisse als das erste und das zweite Messergebnis akzeptiert, wenn ein entsprechender zweiter Aktivierungszustand von einem entsprechenden ersten Aktivierungszustand um eine minimale Spanne abweicht, wobei das erste Messergebnis ($c_{SHT1}$) in Reaktion auf den ersten Aktivierungszustand des Aktors (4) empfangen wird, und wobei das zweite Messergebnis ($c_{SHT2}$) in Reaktion auf den zweiten Aktivierungszustand des Aktuators (4) empfangen wird, und
wobei der Schätzer (9) den Umgebungswert ($c_{amb}$) der Variablen ohne den Beitrag ($c_{mat}$) der einen oder mehreren Komponenten (7) in Abhängigkeit vom ersten Messergebnis ($c_{SHT1}$) und dem zweiten Messergebnis ($c_{SHT2}$) bestimmt.

15. Computerprogramm umfassend Computerprogrammcodemittel zum Bewirken, dass eine Verarbeitungseinheit des Schätzers (9) der Sensoreinheit nach Anspruch 1 die Verfahrensschritte des Schätzers nach Anspruch 14 ausführt.

**Revendications**

1. Unité de détection comprenant

un capteur (3) pour détecter une variable d'un gaz ambiant,
un actionneur (4) pour fournir le gaz ambiant au capteur (3), et
un ou plusieurs composants (7) qui contribuent aux résultats de mesure fournis par le capteur (3),
**caractérisée par** un estimateur (9) conçu pour déterminer une valeur ambiante de la variable sans la contribution du ou des composants (7) en fonction d'un premier résultat de mesure fourni par le capteur (3) en réponse à un premier état d'activation de l'actionneur (4) et d'un deuxième résultat de mesure fourni par le capteur (3) en réponse à un deuxième état d'activation de l'actionneur (4) qui est différent du premier état d'activation,
l'estimateur (9) étant configuré pour surveiller l'état d'activation de l'actionneur (3) et pour accepter deux résultats de mesure fournis par le capteur (3) comme étant le premier et le deuxième résultat de mesure lorsqu'un deuxième état d'activation correspondant diffère d'un premier état d'activation correspondant d'une marge minimale.

2. Unité de détection selon la revendication 1,
dans laquelle l'estimateur (9) est configuré pour surveiller l'état d'activation de l'actionneur (3) et pour accepter les deux résultats de mesure fournis par le capteur (3) comme étant le premier et deuxième résultats de mesure lorsqu'un deuxième état d'activation correspondant diffère d'un premier état d'activation correspondant d'une marge de puissance minimale.

3. Unité de détection selon la revendication 1 ou la revendication 2,
l'estimateur (9) étant conçu de telle sorte qu'il n'accepte les deux résultats de mesure comme premier et deuxième résultats de mesure que si, en outre, un intervalle de temps entre la première mesure correspondante et la deuxième mesure correspondante est inférieur à une valeur seuil.

4. Unité de détection selon la revendication 3, dans laquelle l'intervalle de temps est dimensionné de manière à ce que la valeur ambiante de la variable et la contribution d'un ou de plusieurs composants (7) restent inchangées dans l'intervalle de temps ou qu'elles restent dans une plage définie dans l'intervalle de temps.

5. Unité de détection selon l'une des revendications précédentes,
dans laquelle le premier état d'activation est un état de désactivation de l'actionneur (4) et dans laquelle le deuxième état d'activation est un état d'activation de l'actionneur (4).

6. Unité de détection selon l'une des revendications précédentes 1 à 4,
dans laquelle le premier état d'activation est un état de faible puissance de l'actionneur (4) et dans laquelle le deuxième état d'activation est un état de puissance élevée de l'actionneur (4), dépassant la puissance consommée par l'actionneur (4) dans l'état de faible puissance.

7. Unité de détection selon l'une des revendications précédentes,

dans laquelle l'actionneur (4) est un actionneur qui peut être commandé électriquement au moins dans le premier et le deuxième état d'activation,
l'actionneur comprenant un ou plusieurs d'un ventilateur, d'une pompe ou d'un haut-parleur.

8. Unité de détection selon l'une des revendications précédentes,

dans laquelle le capteur (3) est configuré pour détecter la variable du gaz ambiant, y compris un gaz, un aérosol ou une dispersion,
en particulier, le capteur (3) étant configuré pour détecter la variable du gaz ambiant qui est l'air ambiant entourant l'unité de capteur.

9. Unité de détection selon l'une des revendications précédentes,

comprenant un canal (2) avec une entrée depuis et une sortie vers l'environnement (6),
le capteur (3) étant disposé dans ou sur le canal (2) pour détecter la variable dans le gaz ambiant, et
l'actionneur (4) étant agencé pour guider le gaz ambiant de l'entrée vers la sortie à travers le canal (2), en passant par le capteur (3).

10. Unité de détection selon la revendication 8,

dans laquelle la variable est une température ($T_{amb}$) de l'air ambiant,
le capteur (3) est un capteur de température,
le ou les composants (7) comprennent un composant électronique qui émet de la chaleur en réponse à son alimentation électrique.

11. Unité de détection selon la revendication 8,

dans laquelle la variable est l'humidité ($c_{H2O,amb}$) de l'air ambiant,
le capteur (3) est un capteur d'humidité,
dans laquelle les un ou plusieurs composants (7) comprennent un composant libérant ou absorbant de l'humidité,
en particulier, le ou les composants (7) comprennent un composant d'un boîtier (1) de l'unité de capteur.

12. Unité de détection selon la revendication 8,

la variable étant une concentration de gaz ($c_{amb}$) dans l'air ambiant,
le capteur (3) étant un capteur de gaz sensible au gaz à détecter,
dans laquelle les un ou plusieurs composants (7) comprennent un composant qui libère ou absorbe le gaz auquel le capteur est sensible,
en particulier, les un ou plusieurs composants (7) comprennent un composant d'un boîtier (1) de l'unité de détection,

en particulier dans laquelle la concentration de gaz est une concentration de composés organiques volatils ($c_{tVOC,amb}$), et dans laquelle le capteur de gaz est sensible aux composés organiques volatils,

en particulier dans laquelle le capteur de gaz est un capteur de gaz d'oxyde métallique et dans laquelle l'estimateur est configuré pour déterminer une sensibilité du capteur de gaz d'oxyde métallique en fonction du premier résultat de mesure et du deuxième résultat de mesure.

13. Unité de détection selon l'une des revendications précédentes,

dans laquelle l'estimateur (9) est configuré pour déterminer la valeur d'environnement $c_{amb}$ de la variable sans la contribution $c_{mat}$ du ou des composants (7) sur la base d'un flux de la variable depuis le ou les composants (7) vers le capteur (3) correspondant à un flux de la variable depuis le capteur (3) vers l'environnement (6) pour les premier et deuxième états d'activation de l'actionneur (4), et

en particulier dans laquelle l'estimateur (9) est configuré pour déterminer la valeur d'environnement $c_{amb}$ de la variable sans la contribution $c_{mat}$ des un ou plusieurs composants en résolvant l'équation

$$\alpha_{[1,2]}\,(c_{SHT[1,2]} - c_{amb}) = \beta\,(c_{mat} - c_{SHT[1,2]})$$

où $c_{SHT}$ est un résultat de mesure fourni par le capteur (3), $\alpha$ est un paramètre indiquant le flux de la variable depuis le capteur (3) vers l'environnement (6), et $\beta$ est un paramètre indiquant le flux de la variable depuis le ou les composants (7) vers le capteur (3).

14. Procédé de détermination d'une valeur ambiante ($c_{amb}$) d'une variable dans un gaz dans l'environnement (6) d'une unité de capteur, le procédé comprenant les étapes suivantes :

fournir une unité de détection comprenant un capteur (3) pour détecter la variable, un actionneur (4) pour fournir le gaz ambiant au capteur (3) et un ou plusieurs composants (7) qui contribuent aux résultats de mesure ($c_{SHT}$) fournis par le capteur (3),

**caractérisé par** un estimateur (9) conçu pour déterminer une valeur ambiante de la variable sans la contribution des un ou plusieurs composants (7),

dans lequel l'estimateur (9) surveille un état d'activation de l'actionneur (3) et accepte deux résultats de mesure fournis par le capteur (3) en tant que premier et deuxième résultats de mesure lorsqu'un deuxième état d'activation correspondant s'écarte d'un premier état d'activation correspondant d'une marge minimale, dans lequel le premier résultat de mesure ($c_{SHT1}$) est reçu en réponse au premier état d'activation de l'actionneur (4), et dans lequel le deuxième résultat de mesure ($c_{SHT2}$) est reçu en réponse au deuxième état d'activation de l'actionneur (4) ; et

dans lequel l'estimateur (9) détermine la valeur d'environnement ($c_{amb}$) de la variable sans la contribution ($c_{mat}$) d'un ou plusieurs composants (7) en fonction du premier résultat de mesure ($c_{SHT1}$) et du deuxième résultat de mesure ($c_{SHT2}$).

15. Logiciel comprenant des moyens de code de logiciel pour amener une unité de traitement de l'estimateur (9) de l'unité de détection selon la revendication 1 à exécuter les étapes de procédé de l'estimateur selon la revendication 14.

FIG. 1

FIG. 2

FIG. 3

FIG. 4

FIG. 5

**EP 3 660 498 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20140134053 A1
- US 2012323440 A1
- US 2014355649 A1
- US 2018143084 A1